(19) Europäisches Patentamt
European Patent Office
Office européen des brevets

(11) Publication number: **0 109 859**
**A2**

(12) **EUROPEAN PATENT APPLICATION**

(21) Application number: 83307139.2

(22) Date of filing: 22.11.83

(51) Int. Cl.³: C 12 P 19/42
C 07 H 23/00

(30) Priority: 22.11.82 JP 204905/82
22.11.82 JP 204906/82

(43) Date of publication of application:
30.05.84 Bulletin 84/22

(84) Designated Contracting States:
CH DE FR GB LI

(71) Applicant: NIPPON OIL CO. LTD.
3-12, 1-chome Nishi-Shinbashi
Minato-ku Tokyo(JP)

(72) Inventor: Endo, Noboru
1-4-6 Higashi-Rokugou
Ohta-ku Tokyo(JP)

(72) Inventor: Kojima, Ichiro
4-2-22 Shounan Takatori Takatori
Yokosuka-shi Kanagawa-ken(JP)

(72) Inventor: Oguchi, Yutaka
1-28-17 Higashi-Tamagawa
Setagaya-ku Tokyo(JP)

(74) Representative: Williams, John Francis
J.F. Williams & Co 34 Tavistock Street
London WC2E 7PB(GB)

(54) **Process for purifying and separating vatimin B12.**

(57) A process for the separation of vitamin $B_{12}$ in pure form which comprises bringing a vitamin $B_{12}$-containing liquid having impurities into contact with a divinylbenzene styrene copolymer resin, and causing vitamin $B_{12}$ to be adsorbed to the resin. The vitamin $B_{12}$ thus adsorbed is eluted an eluent which is an aqueous solution containing a compound selected from the group consisting of a lower ketone, a lower ester and a lower ether. The resin may be treated before elution with purified water or an aqueous low-acidity solution.

EP 0 109 859 A2

Croydon Printing Company Ltd.

PROCESS FOR PURIFYING AND SEPARATING VITAMIN $B_{12}$

This invention relates to a process for purifying and separating vitamin $B_{12}$ and more particularly to such a process for the recovery of vitamin $B_{12}$ in pure form from vitamin $B_{12}$-containing liquids having impurities, such as those obtained by a fermentation method, a cellular extraction method and similar methods.

A typical process of the type stated above is known, as disclosed in U.S. Patent 4 383 110, in which vitamin $B_{12}$ of high purity is recovered from a vitamin $B_{12}$-containing liquid by an adsorption-elution operation. In this known process, a starting crude liquid containing vitamin $B_{12}$ and impurities is brought into contact with an adsorbent such as a divinylbenzene/styrene copolymer resin, and vitamin $B_{12}$ is caused to be adsorbed to the adsorbent resin. The vitamin $B_{12}$ adsorbed is then eluted with an eluent such as a hydrous alcohol having an alcohol content of 50% or less, and the active eluate is finally collected.

However, such prior art process is not satisfactory in that vitamin $B_{12}$ can be eluted only at a low rate from the adsorbent resin with a hydrous alcohol, such as 50% aqueous methanol, 40% aqueous ethanol or 20% aqueous isopropanol, resulting in increased consumption of the alcohol to facilitate the eluting operation. Another, more serious problem is that the active eluate thus obtained is diluted in its vitamin $B_{12}$ concentration and, of recrystallised requires further concentration, with added plant and expense.

Where it is desired that vitamin $B_{12}$ be recovered at a rate of say, more than 98% or even maximum 100%, this

dilution of vitamin $B_{12}$ concentration has been found to be extremely disadvantageous. This difficulty is to be eliminated or at least alleviated to obtain industrially and economically satisfactory results.

Another process has also been proposed in which vitamin $B_{12}$ is recovered at high purity from a vitamin $B_{12}$-containing liquid having impurities by an adsorption-washing-elution operation. A starting liquid containing vitamin $B_{12}$ and impurities is brought into contact with an adsorbent such as a divinylbenzene/styrene copolymer resin at a temperature of $10^{o}$ - $30^{o}$, and vitamin $B_{12}$ is caused to be adsorbed to the resin. The resin is then washed with water, or a hydrous alcohol having an alcohol content of less than 20%, or an aqueous low-acidity solution. The latter may comprise a hydrous alcohol such as 20% aqueous methanol,10% aqueous ethanol or 5% aqueous isopropanol, or an aqueous low-acidity solution such as 1% aqueous phoshoric acid, 1% aqueous acetic acid, 1% aqueous boric acid or 0.1% aqueous hydrochloric acid, which may be used in combination depending on the types and quantities of the impurities. Adsorbed vitamin $B_{12}$ is then eluted with a suitable eluent to collect the active eluate.

The prior art process just mentioned has a drawback in that vitamin $B_{12}$ in the active eluate is not satisfactory pure even after the resin which has adsorbed vitamin $B_{12}$ is washed with large amounts of these treating fluids. It is also to be noted that, the hydrous alcohol may cause partial desorption from the resin of the vitamin $B_{12}$.

Yield loss of vitamin $B_{12}$ has been found to be particularly high when the liquids containing vitamin

$B_{12}$ are obtained by a fermentation method or a cellular extraction method, which liquids usually contain relatively large amounts of impurities.

It has now been discovered that certain specific types of eluents such as lower ketones, lower esters and lower ethers enable vitamin $B_{12}$ to be eluted at a high rate with use of smaller amounts of such eluents, that is about 1/5 to 1/15 times that required in the prior art processes. Moreover, since such eluents ensure a 5- to 15- fold increase in vitamin $B_{12}$ concentrations in the active eluate, vitamin $B_{12}$ can be recrystallized, when desired, without the need for any additional concentration treatment. Even with yields as high as 98% - 100%, the purity of vitamin $B_{12}$ is increased 3- to 10- fold, and hence no concentration technique nor any additional purifying means, nor preparatory purifying means is necessary. Consequently, vitamin $B_{12}$ can be separated and recovered at a satisfactory recovery rate of greater than 98%, even sometimes 100%, by simple and easy adsorption and elution operations without any additional or pre-purifying operation.

It has also been discovered that treatment before elution with a treatment fluid such as purified water, being deionized water or distilled water, or aqueous low-acidity solutions, having a temperature of $30^{o}$ - $70^{o}C$, can assist in the elution of vitamin $B_{12}$. Furthermore, these treatment fluids have a selective ability to impart a satisfactory purity to vitamin $B_{12}$ in the active eluate. Vitamin $B_{12}$ of high purity, for example between 30% and 80%, can thus be separated and recovered simply by adsorbing, washing and eluting operations. No additional purification or pre-purification operation is required.

The present invention seeks to provide a process for purifying and separating vitamin $B_{12}$ which is free of the above noted difficulties of the prior art processes and which is efficient, simple and easy.

The present invention further seeks to provide such a process by which vitamin $B_{12}$ can be separated and recovered in optional degrees of purity, yield and recovery with use of such eluents and treating fluids that are specified hereunder without the need for additional purifying and/or pre-purifying operation.

According to the present invention, there is provided a process for the separation of vitamin $B_{12}$, which comprises:

(a) bringing a vitamin $B_{12}$-containing liquid having impurities into contact with a divinylbenzene/styrene copolymer resin;

(b) causing vitamin $B_{12}$ to be adsorbed to said resin;

(c) eluting said adsorbed vitamin $B_{12}$ with an eluent, said eluent being an aqueous solution containing a compound selected from the group consisting of a lower ketone, a lower ester and a lower ether, and

(d) collecting the active eluate.

A resin useful as an adsorbent in the process of this invention is a copolymer obtained by copolymerizing divinylbenzene, styrene and a functional derivitive thereof as repeating units, or a copolymer derived by copolymerizing divinylbenzene, styrene and a functional

unsaturated alkyl ester of an aromatic polycarboxylic acid represented by the formula,

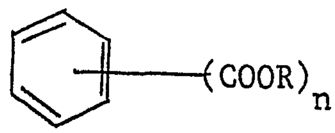

wherein R is a $C_3$ - $C_{10}$ unsaturated alkyl moiety having a carbon-carbon double bond and n is an integer of 2 or 3. This resin is hereinafter referred to as "DST" for brevity.

These copolymers can be produced by copolymerizing the above monomers and ester compound in the presence of a radical polymerization initiator commonly used in the art. The content of the styrene monomer in each of the copolymers is preferably in the range of 30 to 80% by weight, preferably 45 to 70% by weight, based on the total amount of the divinylbenzene and styrene monomers. The content of the unsaturated alkyl ester of an aromatic polycarboxylic acid is preferably in the range of 0.1 to 30% by weight, preferably 1 to 10% by weight, based on the total amount of the divinylbenzene and styrene monomers, and upon the alkyl ester.

The invention will now to illustrated by examples thereof.

A vitamin $B_{12}$-containing liquid having impurities is brought into contact with an adsorbent resin, and vitamin $B_{12}$ is caused to be adsorbed to the resin. Subsequently, the resin which has adsorbed vitamin $B_{12}$ is washed with a treating fluid. The vitamin $B_{12}$ thus adsorbed is eluted with an eluent, and the active eluate is finally collected.

Exemplary crude liquids containing vitamin $B_{12}$ are a liquid obtained by extracting vitamin $B_{12}$ accumulated in the culture broth or cultivated microbial cells which results from aerobically or anaerobically cultivating a known vitamin $B_{12}$-producing microorganism in a nutrient medium, with hot water of 80°C, or a liquid obtained by destroying the cellular membranes of the vitamin $B_{12}$-containing cultivated microbial cells by mechanical or ultrasonic means.

Exemplary vitamin $B_{12}$-producing microorganisms include microorganisms belonging to the genera Propionibacterium, Streptomyces, Arthrobacter, Corynebacterium, Rhodopseudomonas, Mycobacterium and Pseudomonas.

Any suitable contacting means can be used which permits full contact of the vitamin $B_{12}$-containing liquid with the adsorbent resin. For example, a batch method is useful in which the adsorbent is mixed with the vitamin $B_{12}$-containing liquid, and if desired, the admixture is stirred to effect contact of the two. There may also be employed a column chromatographic method which involves filling the resin in a suitable column and allowing the vitamin $B_{12}$-containing liquid to pass through the resin-filled layer.

In the case of the batch method, the pH of the vitamin $B_{12}$-containing liquid is adjusted for example to about 5 - 8, preferably about 7, and added with the adsorbent in a suitable amount, for example, about 1 to about 50 vol/vol. Thereafter, the admixture is gently stirred for a period between 10 minutes and 2 hours, usually for 20 minutes to 1 hour. The adsorption operation may be carried out at room temperature, but is preferably as low as possible, for example, about 10° to about

30°C.

The column chromatographic method can also be carried out by passing the vitamin $B_{12}$-containing liquid through the resin-filled layer under pH and temperature conditions similar to those employed in the batch method.

After the adsorbing step, the resin which has adsorbed vitamin $B_{12}$ is washed with a treatment fluid having a temperature of 30° - 70°C, preferably 40° - 60°C.

Treatment fluids useful for the invention include, for example, purified water such as deionized water and distilled water, and an aqueous low-acidity solution in purified water. Such treatment fluid has a good affinity for the impurities adhering to the surface of the adsorbent resin and these can be selectively stripped from the resin with utmost efficiency and without desorbing vitamin $B_{12}$. In contrast, the treatment fluid employed in the above-mentioned prior art process is poor in its affinity for the impurities.

Acids for the aqueous low-acidity solution useful for the invention include, for example, inorganic acids such as hydrochloric acid, sulphuric acid, phosphoric acid, boric acid and the like, and organic acids such as acetic acid, malic acid and the like. By the term low-acidity solution is meant a solution having an acid concentration of less than 5% by weight, preferably up to 1% by weight.

Impurities include, for example, aliphatic carboxylic acid salts such sodium acetate, sodium propionate, sodium butyrate, sodium valerate and the like, amino acids such as glutamic acid, aspartic acid, proline,

leucine, alanine and the like, saccharoses such as glucose, fructose, ribose, galactose and the like, and nucleic acid salts such as adenine, cytosine, thymine uracil and the like. For example, the solubility of these impurities in water is greater by about 1.3 to 3.0 times at 50°C than at 25°C.

Purified water, which includes distilled water and deionized water containing no water-soluble organic solvent, is also usable. Use of such purified water, unlike the known conventional hydrous alcohols, dispenses with recovering means and gives rise to commercial advantages. Deionized water is preferably 100 x $10^4$ ohm.cm or greater in its resistivity when measured by treatment at an upward rate of 1 ton/day in a tower filled with a conventional ion exchange resin such for example as Amberlite IR-120B or Amberlite IRA-410 (Rohm & Haas Co.).

The treatment fluid may be properly selected depending on the types and amounts of impurities, and the types of adsorbent resins.

After the washing step, adsorbed vitamin $B_{12}$ is eluted with an eluent.

Eluents useful for the invention include aqueous solutions of lower ketones, lower esters and lower ethers, all of which compounds should have 2 to 8 carbon atoms, preferably 3 to 7 carbon atoms. Specific example of such compounds include lower ketones such as acetone, methyl ethyl ketone, diethyl ketone, cyclohexanone, methyl isobutyl ketone and the like, lower esters such as isopropyl acetate, n-propyl acetate and the like, and lower ethers such as tetrahydrofuran, diethyl either, 1,4-diozane,

diisopropyl ether, dimethyl cellosolve, diethyl cellosolve and the like. The concentration of these eluting solutions is usually in the range of 1 to 50% by volume, preferably 5 to 40% by volume.

A choice of eluents also depends on the types and amounts of impurities and the type of adsorbent resins used.

The eluting operation can be performed at room temperature, and heating or cooling is not particularly required. Where found desirable, heating or cooling may be effected, for example, within a temperature range of about 20° to about 60°C.

By the above procedure, the active eluate is obtained, and if desired, recrystallized or otherwise worked up without the need for any concentrating operation.

A fuller understanding of the invention will be had from the following specific examples which are provided for purposes of illustration only and are not to be construed as limiting to the invention.

Example 1

A ground product of microbial cells containing vitamin $B_{12}$ obtained by centrifugally separating a culture broth of a vitamin $B_{12}$-producing organism, Propionibacterium shermanii (IFO 12391), was extracted with water at 80°C to provide a crude liquid containing vitamin $B_{12}$ and impurities (vitamin $B_{12}$ content: 120 ppm).

250cc of the resulting vitamin $B_{12}$-containing liquid was allowed to flow through a column packed with 5cc of a granular DST resin at a flow rate of 0.4cc/minute by

an ascending method to cause vitamin $B_{12}$ to be adsorbed to the resin. Successively, about 30cc of 1% aqueous acetic acid was allowed to flow through the column to cause the impurities to flow into the adsorption waste liquid. KCN-containing water was then allowed to flow through the resin to convert the adsorbed vitamin $B_{12}$ to vitamin $B_{12}$ having the group -CN, i.e. cyanocobalamin (CN-$B_{12}$). Thereafter, 30% aqueous methanol was allowed to flow through the column to elute CN-$B_{12}$. 40cc of the active eluate was collected and dried to give 30mg of CN.$B_{12}$ as powder. The rate of recovery of vitamin $B_{12}$ from the liquid containing vitamin $B_{12}$ and impurities was 100%. The concentration of vitamin $B_{12}$ in the active eluate was 0.75 mg/cc.

The same procedure was repeated except that 21% aqueous dioxane was used to elute CN-$B_{12}$. 8 cc of the active eluate was collected and dried to give 30 mg of CN.$B_{12}$ as powder. The rate of recovery of vitamin $B_{12}$ from the liquid containing vitamin $B_{12}$ and impurities was 100%. The concentration of vitamin $B_{12}$ in the active eluate was 3.75 mg/cc.

It has been found that aqueous acetone is by far more effective as an eluent than aqueous methanol.

Example 2

A ground product of microbial cells containing vitamin $B_{12}$ obtained by centrifugally separating a culture broth of a vitamin $B_{12}$-producing organism, Propionibacterium shermanii (IFO 12391), was extracted with water at 80°C to provide a crude liquid containing vitamin $B_{12}$ and impurities (vitamin $B_{12}$ content: 20 ppm).

800 cc of the resulting vitamin $B_{12}$-containing liquid

was allowed to flow through a column packed with 5 cc of a granular DST resin at a flow rate of 0.4 cc/minute by an ascending method to cause vitamin $B_{12}$ to be adsorbed to the resin. Successively, about 30 cc of 1% aqueous acetic acid was allowed to flow through the column to cause the impurities to flow into the adsorption waste liquid. KCN-containing water was then allowed to flow through the resin to convert the adsorbed vitamin $B_{12}$ to vitamin $B_{12}$ having the group -CN, i.e. cyanocobalamin ($CN-B_{12}$). Thereafter, 15% aqueous isopropanol was allowed to flow through the column to elute $CN-B_{12}$. 80 cc of the active eluate was collected and dried to give 15.7 mg of $CN.B_{12}$ as powder. The rate of recovery of vitamin $B_{12}$ from the liquid containing vitamin $B_{12}$ and impurities was 98%. The concentration of vitamin $B_{12}$ in the active eluate was 0.20 mg/cc.

The same procedure was repeated except that 21% aqueous dioxane was used to elute $CN-B_{12}$. 7.5 cc of the active eluate was collected and dried to give 15.7 mg of $CN.B_{12}$ as powder. The rate of recovery of vitamin $B_{12}$ from the liquid containing vitamin $B_{12}$ and impurities was 98%. The concentration of vitamin $B_{12}$ in the active eluate was 2.09 mg/cc.

It has been found that aqueous dioxane is by far more effective as an eluent than aqueous isopropanol.

Example 3

A ground product of microbial cells containing vitamin $B_{12}$ obtained by centrifugally separating a culture broth of a vitamin $B_{12}$-producing organism, Propionibacterium shermanii (IFO 12391), was extracted with water at 80°C to provide a crude liquid containing vitamin $B_{12}$ and impurities (vitamin $B_{12}$ content: 120

ppm).

250 cc of the resulting vitamin $B_{12}$-containing liquid was allowed to flow through a column packed with 5 cc of a granular DST resin at a flow rate of 0.4 cc/minute by an ascending method to cause vitamin $B_{12}$ to be adsorbed to the resin. Successively, 50 cc of deionized water of 50°C was allowed to flow through the resin to cause the impurities to flow into the adsorption waste liquid. KCN-containing water was then allowed to flow through the resin to convert the adsorbed vitamin $B_{12}$ to vitamin $B_{12}$ having the group -CN, i.e. cyanocobalamin (CN-$B_{12}$). Thereafter, 30% aqueous methanol was allowed to flow through the column to elute CN-$B_{12}$. 40 cc of the active eluate was collected and dried to give CN.$B_{12}$ as powder. The rate of recovery of vitamin $B_{12}$ from the liquid containing vitamin $B_{12}$ and impurities was 100%. The activity rate of the vitamin $B_{12}$ powder separated in pure form was 300 times the level achieved by the powder resulting in crude form from the liquid containing vitamin $B_{12}$ and impurities.

It has been found that hot purified water is effective to treat the resin prior to elution of the adsorbed vitamin $B_{12}$.

<u>Example 4</u>

A ground product of microbial cells containing vitamin $B_{12}$ obtaining by centrifugally separating a culture broth of a vitamin $B_{12}$-producing organism, Propionibacterium shermanii (IFO 12391), was extracted with water at 80°C to provide a crude liquid containing vitamin $B_{12}$ and impurities (vitamin $B_{12}$ content: 40 ppm).

750 cc of the resulting vitamin $B_{12}$-containing liquid was allowed to flow through a column packed with 5cc of a granular DST resin at a flow rate of 0.4 cc/minute by an ascending method to cause vitamin $B_{12}$ to be adsorbed to the resin. Next, 50 cc of 0.5% aqueous acetic acid of 65°C was allowed to flow through the resin to cause the impurities to flow into the adsorption waste liquid. KCN-containing water was then allowed to flow through the resin to convert the adsorbed vitamin $B_{12}$ to vitamin $B_{12}$ having the group -CN, i.e. cyanocobalamin (CN-$B_{12}$). Thereafter, 30% aqueous methanol was allowed to flow through the column to elute CN-$B_{12}$. 40 cc of the active eluate was collected and dried to give CN.$B_{12}$ as powder. The rate of recovery of vitamin $B_{12}$ from the liquid containing vitamin $B_{12}$ and impurities was 98%. The activity ratio of the vitamin $B_{12}$ powder separated in pure form was 300 times the level achieved by the powder resulting in crude form from the liquid containing vitamin $B_{12}$ and impurities.

It has been found that a hot aqueous low-acidity solution is effective to treat the resin prior to elution of the adsorbed vitamin $B_{12}$.

## CLAIMS

1. A process for the separation of vitamin $B_{12}$, which comprises:

(a) bringing a vitamin $B_{12}$-containing liquid having impurities into contact with a divinylbenzene/styrene copolymer resin;

(b) causing vitamin $B_{12}$ to be adsorbed to said resin;

(c) eluting said adsorbed vitamin $B_{12}$ with an eluent, said eluent being an aqueous solution containing a compound selected from the group consisting of a lower ketone, a lower ester and a lower ether, and

(d) collecting the active eluate.

2. The process according to claim 1 wherein said resin is selected from the group consisting of a copolymer resin of divinylbenzene/styrene/a functional derivative thereof and a copolymer of divinylbenzene/styrene/a functional derivative thereof/an unsaturated alkyl ester of an aromatic polycarboxylic acid represented by the formula,

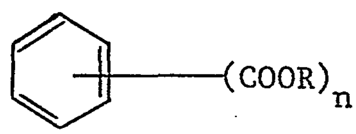

wherein R is a $C_3$-$C_{10}$ unsaturated alkyl moiety having a carbon-carbon double bond and n is an integer of 2 or 3.

3. The process according to claim 1 wherein said vitamin $B_{12}$-containing liquid has a pH of 5 to 8.

4. The process according to claim 1 wherein prior to step (c), said resin is treated with a treatment fluid having a temperature of from 30° - 70°C.

5. The process according to claim 4 wherein said treating fluid is a purified water or an aqueous low-acidity solution in purified water.

6. The process according to claim 5 wherein said purified water is selected from the group consisting of distilled water and deionized water, and said low-acidity solution contains an inorganic or organic acid selected from the group consisting of hydrochloric acid, sulphuric acid, phosphoric acid, acetic acid and malic acid.

7. The process according to claim 6 wherein said low-acidity solution has an acid concentration of up to 5% by weight.

8. The process according to claim 1 wherein said lower ketone is selected from the group consisting of acetone, methyl ethyl ketone, diethyl ketone, cyclohexanone and methyl isobutyl ketone, said lower ester is selected from the group consisting of isopropyl acetate and n-propyl acetate, and said lower ether is selected from the group consisting of tetrahydrofuran, diethyl ether, 1,1-dioxane, diisopropyl ether, dimethyl cellosolve and diethyl cellosolve.

9. The process according to claim 1 wherein said eluting solution has a concentration of 1 to 50% by volume.

10. A process for the separating of vitamin $B_{12}$ substantially as herein described in any of Examples 1 to 4.